Europäisches Patentamt

European Patent Office

Office européen des brevets

(19)

(11) Numéro de publication : **0 191 263**
**B1**

(12) **FASCICULE DE BREVET EUROPÉEN**

(45) Date de publication du fascicule du brevet :
**07.06.89**

(21) Numéro de dépôt : **85440007.4**

(22) Date de dépôt : **11.02.85**

(51) Int. Cl.⁴ : **A 61 M   7/00**

(54) Installation chirurgicale d'irrigation à flux continu de solution stérile.

(73) Titulaire : **Letartre, Thierry**
**6, Allée de la Cerisaie**
**F-59700 Marcq-En-Baroeul (Nord) (FR)**

(72) Inventeur : **Letartre, Thierry**
**6, Allée de la Cerisaie**
**F-59700 Marcq-En-Baroeul (Nord) (FR)**

(74) Mandataire : **Lepage, Jean-Pierre**
**Cabinet Lepage & Aubertin Innovations et Presta-**
**tions 23/25, rue Nicolas Leblanc B.P. 1069**
**F-59011 Lille Cédex 1 (Nord) (FR)**

(43) Date de publication de la demande :
**20.08.86 Bulletin 86/34**

(45) Mention de la délivrance du brevet :
**07.06.89 Bulletin 89/23**

(84) Etats contractants désignés :
**BE CH DE FR GB IT LI LU NL**

(56) Documents cités :
**FR-A- 2 021 621**
**US-A- 3 044 465**
**US-A- 4 253 457**

Jouve, 18, rue St-Denis, 75001 Paris, France

## Description

L'invention est relative à une installation chirurgicale d'irrigation à flux continu de solution stérile. Elle trouvera notamment son application dans le milieu médical pour les cystoscopies, les résections transurétrales ou les irrigations post-opératoires.

Avec le développement des fibres optiques, l'endoscopie s'est fortement implantée dans les techniques opératoires modernes.

Ainsi, alors qu'auparavant l'ablation de la prostate nécessitait une ouverture de l'abdomen du patient, actuellement, il est possible de procéder à la résection en tout ou partie de la prostate par voie interne.

La résection de la prostate consiste à produire une désagrégation des parties de la prostate qui doivent être éliminées et d'évacuer les particules de prostate détruites par l'intermédiaire d'un flux continu d'eau stérile qui irrigue la zone opérée.

La conduite de l'opération est menée par le chirurgien qui observe la zone opératoire à l'aide d'une fibre optique introduite dans la vessie du patient à travers les voies urinaires, ce même canal servant également au passage du résecteur d'amenée d'eau stérile et du bistouri électrique.

Durant toute l'opération, le résecteur permet de rincer et d'irriguer la prostate et assure également l'évacuation des particules par circulation d'eau stérile.

Il est certain que la solution d'irrigation qui est généralement de l'eau stérile doit présenter des propriétés particulières adaptées aux opérations effectuées.

En particulier, la solution d'irrigation doit être exempte de germes afin de ne provoquer aucune contamination. De plus, il a été remarqué qu'il ne suffit pas de détruire les bactéries mais encore faut-il éliminer les cadavres de ces derniers. Cette propriété peut être obtenue en utilisant des filtres apyrogène, qui évitent les poussées de fièvre chez le patient.

En outre, la solution d'irrigation doit être isotonique ou non hémolysante c'est-à-dire ne pas pouvoir pénétrer dans les veines du patient et y détruire les globules rouges. Deux additifs sont généralement utilisés pour répondre à ce besoin, il s'agit soit du glucose ou soit du chlorure de sodium.

D'autres additifs peuvent également être introduits dans la solution d'irrigation pour par exemple faciliter la coagulation.

Notamment pour des problèmes d'anesthésie, il est également généralement demandé que la solution d'irrigation présente une température de 30 à 35 °C en sortie de résecteur afin de n'induire aucun trouble chez le patient opéré.

Par ailleurs, puisque dans le cas de la résection de la prostate, la solution d'irrigation est introduite au niveau de la vessie du patient, il est impératif de contrôler avec une grande précision la pression de la solution d'irrigation en sortie de résecteur.

Actuellement, les installations chirurgicales d'irrigation à flux continu d'eau stérile se présentent sous la forme de vase d'une contenance de 3 à 4 litres dans lequel est versée de l'eau additionnée de glucose ou chlorure de sodium et recouverte par une rampe UV de stérilisation. Le résecteur est disposé en aval du dit vase et des filtres intermédiaires peuvent être utilisés pour obtenir une solution d'irrigation adéquate.

Ce type d'installation souffre de nombreux inconvénients en particulier parce qu'il nécessite une préparation de la solution d'irrigation sur le site opératoire et que cela demande un personnel qualifié. De plus, les garanties d'antisepsie d'une telle installation sont précaires.

Aussi, pour perfectionner le type d'installation précédent il a été développé la production de solution stérile sous forme de poche souple utilisable telle que des cartouches qui sont interchangées au fur et à mesure des besoins.

Ce type d'installation bien que présentant un gros progrès vis-à-vis du type précédent, comporte encore de nombreux inconvénients. La capacité d'une poche souple contenant la solution d'irrigation est d'environ 3 litres, or les besoins en solution pour une opération sont compris entre 10 et 30 litres selon les interventions. Il s'ensuit que durant l'opération, il est nécessaire d'échanger entre 3 et 10 fois les poches souples de solution stérile utilisées, ce qui constitue une manipulation incommodante.

Par ailleurs, il est nécessaire de prévoir le stockage des poches de solution stérile qui occupe un volume important et qui, par conséquent, présente un coût de revient relativement élevé.

Il est également souhaitable de réchauffer jusqu'à 30 ou 35 °C la solution d'irrigation et par conséquent il faut utiliser un dispositif à bain marie pour réchauffer les poches souples. Il est connu que de telles installations sont particulièrement désagréables à utiliser.

Il faut également tenir une comptabilité du nombre de poches utilisées afin de totaliser la quantité de solution d'irrigation introduite dans le patient et vérifier que cette quantité corresponde bien à celle recueillie dans un bassin gradué à cet effet.

Il est à noter qu'il est connu du document FR-A-2.021.621 une installation chirurgicale d'irrigation à flux continu de solution stérile qui permet de délivrer une solution d'irrigation isotonique non hémolysante à pression contrôlée à partir d'un réseau d'eau courante.

Selon ce document, l'installation comporte : un circuit de dépressurisation de l'eau, une cellule de filtrage, un poste d'addition iso-osmolaire de glucose, ainsi qu'un récipient irrigateur autonome qui assure l'alimentation d'un résecteur sous forme pression réglable.

Cependant, une telle installation n'est pas sans présenter certains inconvénients notamment au niveau de la formation du mélange de l'eau courante avec le glucose. De plus, l'installation est livrée à elle-même et aucun contrôle n'est opéré.

Cela étant, il est également connu du document US-A-3.044.465 une installation chirurgicale d'irrigation à flux continu de solution stérile dans laquelle on effectue un mélange entre de l'eau et un produit thérapeutique prédéterminé, ce à l'aide d'une pompe non contrôlée.

Une telle technique permet d'injecter dans l'eau la solution thérapeutique de façon plus efficace que dans l'installation du document français précité. Néanmoins, cette installation n'est en aucun cas contrôlée et ne permet pas notamment de fonctionner en toute sécurité pour le patient.

Le but principal de la présente invention est de présenter une installation chirurgicale d'irrigation à flux continu de solution stérile qui puisse directement être alimentée à partir d'eau courante sous pression et qui permette de pallier les inconvénients des dispositifs connus.

Il en résulte une simplicité d'utilisation considérable vis-à-vis de la technique actuelle puisqu'il n'est plus nécessaire de procéder à un quelconque remplissage ou échange des poches souples de solution stérile durant l'opération. L'eau courante sous pression utilisée pourra directement être obtenue à partir du circuit urbain de distribution d'eau.

La présente invention présente toutes les garanties d'aseptie nécessaires puisqu'elle a été conçue de sorte à ce que la partie principale c'est-à-dire le récipient irrigateur soit autonome et puisse être stérilisé à l'autoclave.

Pour plus de sécurité, l'installation d'irrigation de la présente invention comporte un débitmètre à affichage numérique qui indique à tout moment la quantité totale de solution délivrée par le résecteur. En comparant cette quantité avec celle récupérée dans le bassin gradué utilise, on peut déterminer, de façon précise, la quantité de solution résiduelle restant dans le patient. Toute accumulation peut par conséquent être immédiatement détectée.

Selon la présente invention, l'installation chirurgicale d'irrigation à flux continu de solution stérile, destinée notamment en urologie à assurer l'évacuation des résidus d'organes dans les résections transurétrales, qui permet de délivrer une solution d'irrigation isotonique non hémolysante à pression contrôlée à partir d'un réseau d'eau courante, ladite installation comprenant :

— un circuit de dépressurisation de l'eau,

— un poste d'addition iso-osmolaire de concentré non hémolysant, présentant des moyens pour ajouter dans l'eau courante le dit concentré pour former la dite solution d'irrigation isotonique non hémolysante,

— une cellule de filtrage de la solution eau + concentré,

— un récipient irrigateur autonome, destiné à contenir la dite solution d'irrigation, qui assure l'alimentation du résecteur sous faible pression réglable, caractérisée par le fait qu'elle comporte :

— une cellule de rétention des pyrogènes,

— une pompe doseuse péristaltique apte à injecter le dit concentré dans le circuit d'eau courante,

— un débitmètre, contrôlant la dite pompe, afin d'injecter le dit concentré en une quantité correspondante au débit d'eau transitant dans la dite installation et proportionnelle au dit débit d'eau,

— un afficheur, précédé d'un intégrateur coopérant avec le débitmètre, apte à indiquer la quantité totale de solution ayant transité dans le débitmètre et un récipient gradué qui permet de déterminer la quantité de solution récupérée du patient.

L'invention sera mieux comprise à la lecture de la description suivante accompagnée d'un dessin en annexe qui illustre schématiquement le diagramme de fonctionnement de l'installation d'irrigation de la présente invention.

L'installation d'irrigation chirurgicale à flux continu de solution stérile de la présente invention trouve son application notamment pour la cystocopie, les résections transurétrales ou les irrigations post-opératoires.

L'exemple choisi pour illustrer la présente invention est la résection de la prostate, toutefois, il est évident que d'autres applications sont également envisageables.

L'installation d'irrigation de la présente invention permet de délivrer une solution d'irrigation stérile c'est-à-dire ne contenant aucun germe actif et également sans pyrogène c'est-à-dire de cadavres de bactéries. En outre, la solution est isotonique non hémolysante c'est-à-dire non destructrice de globules rouges.

L'installation de la présente invention est destinée à prendre comme source, l'eau courante pressurisée généralement distribuée par le réseau urbain. Cette caractéristique présente une facilité d'utilisation considérable puisque la quantité d'eau ainsi disponible est quasi illimitée.

Bien entendu, l'eau courante disponible doit être traitée pour former une solution d'irrigation.

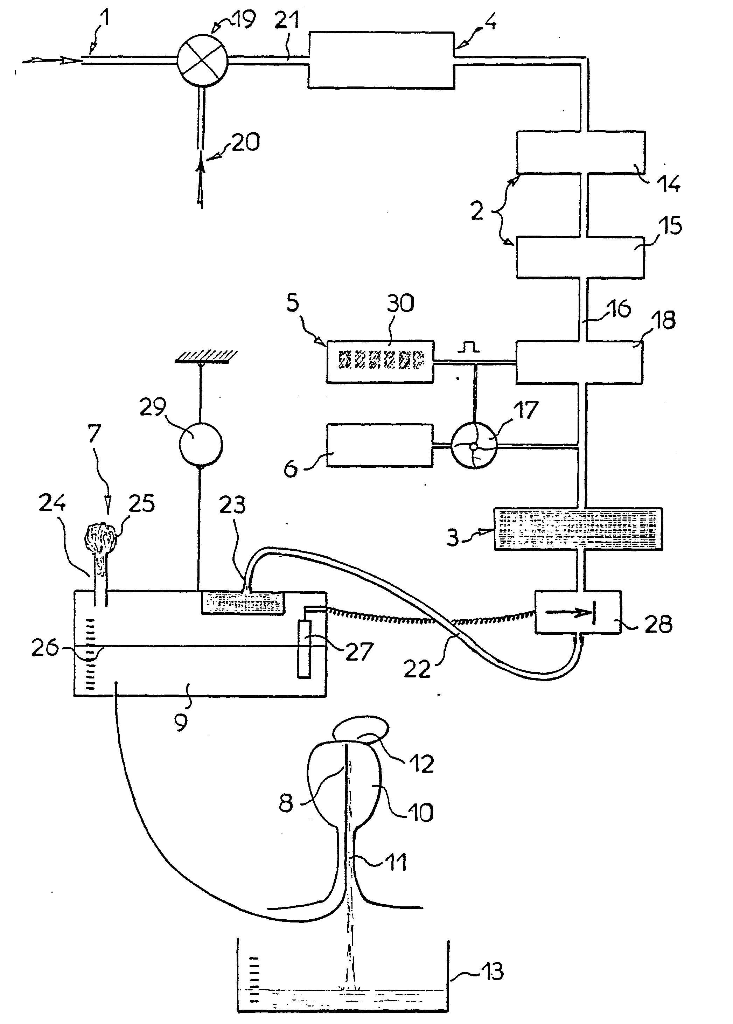

L'installation d'irrigation illustrée sur la figure en annexe comprend un circuit d'amenée d'eau 1 courante qui pourra, par exemple, être prélevée sur le réseau de distribution urbain. Il s'agit d'une eau pressurisée et par conséquent inapte sur ce plan à former une solution d'irrigation. L'installation comprend un circuit 2 de dépressurisation qui permet ainsi d'obtenir en sortie du dit circuit une eau sous très faible pression.

L'installation comprend également une cellule de filtrage 3 et de rétention des pyrogènes 4. Le rôle de cette cellule est de retenir les pyrogènes c'est-à-dire les cadavres de bactéries et également de retenir en grande partie les germes véhiculés par l'eau courante.

L'installation comprend également un poste 5 d'addition iso-osmolaire de concentré non hémolysant. Ce poste permet d'injecter dans l'eau courante les additifs nécessaires pour former une solution d'irrigation présentant des propriétés non hémolysantes, isotoniques.

L'injection est réalisée à partir de concentrés 6 et proportionnelle au débit d'eau pour former une solution d'irrigation présentant une teneur en additif constante quel que soit le débit.

Il sera de préférence utilisé une solution de glucose pour former le concentré au lieu de chlorure de sodium car ce dernier présente des propriétés de conductibilité électrique difficilement compatibles avec l'utilisation de bistouris électriques.

Enfin l'installation comprend un récipient irrigateur autonome 7 qui assure l'alimentation du résecteur 8 sous faible pression ajustable.

Cette propriété d'indépendance du récipient irrigateur 7 vis-à-vis du reste de l'installation est importante car il s'agit du cœur de l'installation qui contient la solution d'irrigation 9 définitive bénéficiant de toutes les propriétés nécessaires à la résection. Par conséquent, il s'agit d'une pièce qui doit pouvoir subir des stérilisations à l'autoclave et qui doit pouvoir être interchangée facilement.

Dans l'exemple choisi, le résecteur 8 est introduit dans la vessie 10 du patient par le canal des voies urinaires 11 pour assurer l'irrigation de la zone de la prostate 12 opérée.

La solution utilisée pour assurer l'évacuation des résidus de prostate est récupérée dans un bassin gradué 13 qui permet ainsi de déterminer la quantité de solution recueillie.

Sur la figure, il n'est pas représenté le bistouri électrique et la fibre endoscopique qui permet au chirurgien d'opérer la prostate 12.

Selon un mode préférentiel de réalisation de la présente invention, le circuit de dépressurisation 2 est composé d'un double étage schématisé par le détenteur 1-14 et le détenteur 2-15 sur la figure. Le double étage permet respectivement de ramener la pression de l'eau courante à sensiblement 2 bars et 0,6 bar en sortie 16 du circuit de dépressurisation de l'eau.

La cellule de filtrage 3 et de rétention des pyrogènes 4 comprend en ce qui concerne le pré-filtre apyrogène une cartouche à 1,5 micron destinée à retenir les pyrogènes et le filtre 3 comprend une cartouche à 0,3 micron.

De préférence, le pré-filtre apyrogène 4 est placé en amont du circuit de dépressurisation 2, par contre, le filtre 4 est placé en aval du dit circuit de dépressurisation 2.

Le poste d'addition iso-osmolaire 5 comprend une pompe doseuse péristaltique 17 commandée par un débitmètre 18 qui permet d'injecter du concentré 6 en quantité correspondante au débit d'eau transitant dans l'installation d'irrigation. De la sorte, il y a formation d'une solution d'irrigation isotonique non hémolysante à partir d'eau courante 1 et de concentré 6, toutefois, seule la solution concentrée 6 est stockée au niveau de l'installation d'irrigation qui, par conséquent, peut présenter un volume réduit.

De préférence, la solution d'irrigation présente une température avoisinant 30 à 35 °C. Pour atteindre cette température, on équipe avantageusement l'installation d'irrigation de la présente invention d'un mitigeur 19 placé à l'entrée de l'installation et qui est alimenté en eau chaude 20. Le mitigeur permet de délivrer à sa sortie 21 une eau tempérée à partir de l'eau courante 1 et de l'eau chaude 20.

Selon la présente invention, le récipient irrigateur 7 est relié au reste de l'installation par un tuyau souple 22 d'amenée de la solution d'irrigation pré-filtrée, isotonique, non hémolysante, à sensiblement 35 °C.

Le récipient irrigateur 7 est de préférence formé par des parois rigides et transparentes. Il est réalisé dans une matière stérilisable et comprend à l'entrée 23 de la solution d'irrigation un filtre à cartouche à 0,2 micron chargée à-pyrogène pour stériliser la solution. Un évent 24 met en communication le volume intérieur du récipient irrigateur 7 avec l'atmosphère afin d'obtenir un équilibrage des pressions. L'évent 24 est muni d'un filtre à cartouche 25 pour stériliser l'air transitant par l'évent. Une cartouche à 0,4 micron donne de bons résultats.

Le niveau 26 de la solution 9 dans le récipient irrigateur 7 est contrôlé à l'aide d'une sonde 27 qui agit sur une vanne 28 afin d'établir un niveau sensiblement constant de solution 9 dans le récipient 7.

La sonde de niveau 27 pourra être par exemple du type électronique et commander électriquement la vanne 28 disposée en sortie de cellule de filtrage 3.

Cette conception de l'installation permet d'obtenir une solution 9 dans le récipient irrigateur 7 qui ne soit uniquement soumis qu'à la pression atmosphérique, il s'agit par conséquent d'une dépressurisation totale de l'eau courante 1 initiale.

Pour ajuster la pression en sortie de résecteur 8, il est commode d'utiliser un support 29 du récipient 7 ajustable en hauteur. Divers types de supports peuvent être utilisés tels qu'un pied à sérum ou un équilibreur balancier accroché au plafond.

Le contrôle de la quantité de solution d'irrigation introduite dans le patient pourra avantageusement être réalisé à l'aide d'un affichage 30 précédé d'un intégrateur qui permettra d'indiquer la quantité totale ayant transité dans le débitmètre 18. Le bassin de récupération 13 étant gradué, la détermination de toute accumulation de solution dans le patient pourra être immédiatement détectée. Une telle installation permet de produire une quantité de solution d'irrigation adaptée aux besoins et le récipient irrigateur 7 sera stérilisé à l'autoclave entre deux utilisations et les filtres 23 et 24 seront remplacés sensiblement toutes les 30 stérilisations.

## Revendications

1. Installation chirurgicale d'irrigation à flux continu de solution stérile, destinée notamment en urologie à assurer l'évacuation des résidus d'organes dans les résections transurétrales, qui permet de délivrer une solution d'irrigation isotonique non hémolysante à pression contrôlée à partir d'un réseau d'eau courante, la dite installation comprenant :

— un circuit de dépressurisation de l'eau (2),

— un poste (5) d'addition iso-osmolaire de concentré (6) non hémolysant, présentant des moyens pour ajouter dans l'eau courante (1) le dit concentré (6) pour former la dite solution d'irrigation isotonique non hémolysante,

— une cellule de filtrage (3) de la solution eau + concentré,

— un récipient (7) irrigateur autonome, destiné à contenir la dite solution d'irrigation (9), qui assure l'alimentation du résecteur (8) sous faible pression réglable, caractérisée par le fait qu'elle comporte :

— une cellule de rétention des pyrogènes (4),

— une pompe doseuse (17) péristaltique apte à injecter le dit concentré (6) dans le circuit d'eau courante (1),

— un débitmètre (18), contrôlant la dite pompe (17), afin d'injecter le dit concentré (6) en une quantité correspondante au débit d'eau (1) transitant dans la dite installation et proportionnelle au dit débit d'eau,

— un afficheur (30), précédé d'un intégrateur coopérant avec le débitmètre (18), apte à indiquer la quantité totale de solution ayant transité dans le débitmètre et un récipient gradué (13) qui permet de déterminer la quantité de solution récupérée du patient.

2. Installation d'irrigation selon la revendication 1, caractérisée par le fait que le circuit (2) de dépressurisation est à double étage qui permettent respectivement de ramener la pression de l'eau à sensiblement 2 bars et 0,6 bar.

3. Installation d'irrigation selon la revendication 1 caractérisée par le fait que la cellule de filtrage (3) et de rétention des pyrogènes (4) comprend un pré-filtre à cartouche à 1,5 micron destinée à retenir les pyrogènes, et un filtre à cartouche à 0,3 micron.

4. Installation d'irrigation selon la revendication 1, caractérisée par le fait qu'en amont de la cellule de filtrage (3, 4) un mitigeur (19) alimenté en eau chaude (20) introduit cette dernière dans le circuit d'eau courante (1) de sorte à obtenir une eau tempérée (21) en sortie de mitigeur (19).

5. Installation d'irrigation selon la revendication 1, caractérisée par le fait que le récipient (7) irrigateur est formé de trois parois rigides stérilisables et qu'il comprend un évent (24) débouchant à l'air libre, le dit évent (24) comportant des moyens de stérilisation (25) de l'air entrant dans le dit récipient (7).

6. Installation d'irrigation selon la revendication 1, caractérisée par le fait que le récipient irrigateur (7) comporte à l'entrée (23) un filtre à cartouche chargé apyrogène.

7. Installation d'irrigation selon la revendication 1, caractérisée par le fait que le récipient (7) comporte un circuit de contrôle (27) du niveau (26) de solution (9) et qui assure le remplissage du dit récipient (7) par commande d'une vanne (28).

## Claims

1. Surgical installation for continuous flow irrigation with a sterile solution, intended in particular in urology to ensure the evacuation of the residues of organs in transurethral resections, that enables an isotonic, non-haemolyzing irrigation solution to be delivered at controlled pressure from a running water network :

— a water depressurizing circuit (2),

— a station (5) for the iso-osmolar addition of non-haemolyzing concentrate (6), having means for adding the said concentrate (6) to the running water (1) to form the said isotonic, non-haemolyzing irrigation solution,

— a cell (3) for filtering the water + concentrate solution,

— an independent irrigating recipient (7), designed to contain the said irrigation solution (9), which ensures the supply of the resector (8) at an adjustable low pressure, characterized by the fact that it comprises :

— a pyrogen retention cell (4),

— a peristaltic proportioning pump (17) suitable for injecting the said concentrate (6) into the running water circuit (1),

— a flowmeter (18), monitoring the said pump (17), in order to inject the said concentrate (6) in a quantity corresponding to the flow of water (1) passing through the said installation and proportional to the said flow of water,

— a display means (30), preceded by an integrator cooperating the with flowmeter (18), suitable for indicating the total quantity of solution that has passed through the flowmeter and a graduated recipient (13) that enables the quantity of solution recovered from the patient to be determined.

2. Irrigation installation according to claim 1, characterized by the fact that the depressurizing circuit (2) is in two stages that enable the pressure of the water to be reduced to substantially 2 bars and 0.6 bar.

3. Irrigation installation according to claim 1, characterized by the fact that the filtering (3) and pyrogen retention (4) cell includes a 1.5 micron cartridge pre-filter designed to retain the pyrogens, and a 0.3 micron cartridge filter.

4. Irrigation installation according to claim 1, characterized by the fact that, upstream of the filtering cell (3, 4), a mixing valve (19) supplied with hot water (20) introduces the latter into the running water circuit (1) so as to obtain lukewarm water (21) at the output of the mixing valve (19).

5. Irrigation installation according to claim 1, characterized by the fact that the irrigating recipient (7) is formed by three sterilizable rigid walls and that it comprises a vent (24) emerging in the ambient air, the said vent (24) comprising means (25) for sterilizing the air entering the said recipient (7).

6. Irrigation installation according to claim 1, characterized by the fact that the irrigating recipient (7) comprises at the input (23) an a-pyrogen loaded cartridge filter.

7. Irrigation installation according to claim 1, characterized by the fact that the recipient (7) comprises a circuit (27) for monitoring the level (26) of solution (9) and that ensures the filling of the said recipient (7) under the control of a valve (28).

**Patentansprüche**

1. Chirurgische Irrigationsvorrichtung mit kontinuierlich strömender, steriler Lösung, insbesondere dazu bestimmt, in der Urologie bei transurenalen Resektionen die Organrückstände auszuspülen, mittels der es möglich ist, bei Anschluß an ein Leitungswassernetz eine nicht hämolysierende, isotonische Irrigationslösung mit gesteuertem Druck abzugehen, aus :

— einer Einrichtung (2) zur Reduzierung des Wasserdrucks ;

— einer Station (5) zur isoosmolaren Zugabe von nicht hämolysierendem Konzentrat (6), die Mittel aufweist, um das besagte Konzentrat (6) in das Leitungswasser (1) zu geben, wodurch die besagte nicht hämolysierende, isotonische Irrigationslösung erhalten wird ;

— einer Zelle (3) zur Filtration der Lösung aus Wasser und Konzentrat ;

— einem autonomen Irrigationsbehälter (7), der für die Aufnahme der besagten Irrigationslösung (9) bestimmt ist, und der die Versorgung des Resektors (8) bei niedrigem, einstellbarem Druck sicherstellt ; dadurch gekennzeichnet, daß sie aufweist :

— eine Zelle (4) zum Zurückhalten der Pyrogene ;

— eine peristaltische Dosierpumpe (17), mit der das besagte Konzentrat (6) in das Leitungswasser (1) eingespritzt werden kann ;

— einen Strömungsmesser (18), über den die besagte Pumpe (17) so gesteuert wird, daß sie das besagte Konzentrat (6) in einer zu der Strömungsrate des Leitungswassers (1) proportionalen Menge einspritzt ;

— eine Anzeigevorrichtung (30) mit vorgeschaltetem Integrator, der mit dem Strömungsmesser (18) zusammenwirkt, und der die gesamte Lösungsmenge anzeigt, die durch den Strömungsmesser hindurchgeflossen ist ;

— und einen Behälter (13) mit Meßskala, mit dem die bei dem Patienten zurückgewonnene Lösungsmenge bestimmt werden kann.

2. Irrigationsvorrichtung gemäß Anspruch 1, dadurch gekennzeichnet, daß die Einrichtung (2) zur Druckreduzierung zwei Stufen aufweist, die gestatten, den Druck des Wassers auf ungefähr 2 bar bzw. 0,6 bar zu reduzieren.

3. Irrigationsvorrichtung gemäß Anspruch 1, dadurch gekennzeichnet, daß die Filtrationszelle (3), und die Zelle (4) zur Zurückhaltung der Pyrogene ein Patronen-Vorfilter mit 1,5 μm Porengröße zum Zurückhalten der Pyrogene, und eine Patronenfilter mit 0,3 μm Porengröße aufweisen.

4. Irrigationsvorrichtung gemäß Anspruch 1, dadurch gekennzeichnet, daß stromaufwärts von -den Filtrationszellen (3, 4) ein mit warmem Wasser (20) versorgtes Mischventil (19) dieses warme Wasser (20) dem Leitungswasser (1) zumischt, so daß am Ausgang des Mischventils (19) lauwarmes Wasser (21) erhalten wird.

5. Irrigationsvorrichtung gemäß Anspruch 1, dadurch gekennzeichnet, daß der Irrigationsbehälter (7) von drei sterilisierbaren starren Wänden gebildet wird, und daß er eine nach draußen führende Lüftungsöffnung (24) aufweist, die mit Mitteln (25) zur Sterilisierung der in den besagten Behälter (7) einströmenden Luft versehen ist.

6. Irrigationsvorrichtung gemäß Anspruch 1, dadurch gekennzeichnet, daß der Irrigationsbehälter (7) an dem Eingang (23) ein apyrogenes Patronenfilter aufweist.

7. Irrigationsvorrichtung gemäß Anspruch 1, dadurch gekennzeichnet, daß der Behälter (7) einen Kreis (27) zur Steuerung des Niveaus (26) der Lösung (9) aufweist, der die Füllung des besagten Behälters (7) über ein Ventil (28) steuert.

1